# EUROPEAN PATENT APPLICATION

(11) **EP 4 830 755 A1**
(43) Date of publication of application: **28.10.2026**
(21) Application number: 26173599.7
(22) Date of filing: 21.04.2026
(51) Int. Cl.: A61M 16/20, A61M 16/00, A61M 16/18, G01F 25/00, A61M 16/10

(54) **SYSTEMS AND METHODS FOR CALIBRATING AN ANESTHESIA VALVE**

(30) Priority: 25.04.2025 US 202519190451
(71) Applicant: GE Precision Healthcare LLC, Waukesha, WI 53188 (US)
(72) Inventor: GUPTA, Nikhil, 560066 Bengaluru (IN); VARGHESE, Sanju Alias Kooran, 560066 Bengaluru (IN); BRAUER, Kimberly A, Madison, 53718-6704 (US); BAUM, Bradley, Madison, 53718-6704 (US)
(74) Representative: Kilburn & Strode LLP

(57) **Abstract**

Various methods and systems are provided for an anesthetic vaporizer. As an example, a method includes calibrating a liftoff current of a proportional valve arranged between a vaporization chamber and a mixing manifold of an anesthetic vaporizer via a controller following a standby mode and prior to a therapy mode, wherein the liftoff current is a current supplied to actuate the proportional valve from a closed position to an open position.

## Description

### FIELD

Embodiments of the subject matter disclosed herein relate to calibrating an anesthesia valve lift-off.

### BACKGROUND

Anesthesia vaporizer systems precisely deliver anesthetic agent to anesthetic machines by precisely controlling the opening of proportional valves. The proportional valve may be operated at a range of currents based on a desired flow rate. An opening current of the valve may be close to an operating current range of the proportional valve at lower flow rates. The valve opening may be slower when a lower flow rate is requested to avoid overshooting a requested anesthesia amount.

### BRIEF DESCRIPTION

In one example, a method calibrating a liftoff current of a proportional valve arranged between a vaporization chamber and a mixing manifold of an anesthetic vaporizer via a controller following a standby mode and prior to a therapy mode, wherein the liftoff current is a current supplied to actuate the proportional valve from a closed position to an open position. In this way, an opening time of the proportional valve may be reduced and more accurately controlled.

It should be understood that the brief description above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

The present disclosure will be better understood from reading the following description of non-limiting embodiments, with reference to the attached drawings, wherein below:
FIG. 1 shows a schematic of a vaporizer.
FIG. 2 shows a method for determining if calibration conditions are met.
FIG. 3 shows a method for determining a mode of operation of an anesthetic vaporizer.
FIG. 4 shows a method for initiating a calibration mode of a valve of the anesthetic vaporizer.
FIG. 5 shows a method for transitioning from the calibration mode to a therapy mode.
FIG. 6 shows a method for determining if one or more conditions for deactivating the calibration mode is met.
FIG. 7 shows an operating sequence of the anesthetic vaporizer.
FIG. 8 shows a method tied to hardware and software of the anesthetic vaporizer for executing the calibration mode.
FIG. 9 shows graph illustrating a calibration of a liftoff current of a valve of the anesthetic vaporizer based on a first anesthetic agent.
FIG. 10 shows a graph illustrating a calibration of a liftoff current of a valve of the anesthetic vaporizer based on a second anesthetic agent.
FIG. 11 shows a graph illustrating a calibration of a liftoff current of a valve of the anesthetic vaporizer based on a third anesthetic agent.
FIG. 12 shows a schematic of an anesthetic vaporizer.

### DETAILED DESCRIPTION

The following description relates to systems and methods for an anesthesia system. Conventionally, anesthesia systems include a control valve and a proportional valve. During operation, the proportional valve is operated to open an anesthetic agent path to allow the anesthetic agent to mix with a gas in a mixing manifold. At certain flow rates of the anesthetic agent, such as lower flow rates, a current used to open the proportional valve may be within a threshold range of a current used to hold the proportional valve open. At these flow rates, the system may slowly open the proportional valve to avoid overshooting (e.g., providing more anesthetic agent than desired). However, anesthesia delivery may be time-sensitive and the slow opening of the proportional valve may impact patient outcomes.

The inventors hereby recognize the disadvantages discussed above and hereby attempt to address these disadvantages by calibrating a proportional valve of the anesthesia system. By calibrating the proportional valve following a standby mode and prior to a therapy mode, the proportional valve may be desirably opened with a current liftoff that does not overshoot a target anesthetic concentration at lower flow rates.

As an example, methods and systems included herein are related to controlling an anesthetic agent delivery system by precisely determining and controlling the opening current of a proportional valve in an anesthesia vaporizer. In existing systems, when the commanded valve working range is close to the valve opening current, it becomes challenging to achieve desired performance in agent delivery control, particularly at low flow rates. This challenge arises because each valve has different opening characteristics due to manufacturing variations.

The present disclosure at least partially solves the above-described problems by implementing a "liftoff finder routine", referred to herein interchangeably as a calibration mode, that decouples the valve opening control from the agent delivery control. The routine determines the specific opening current desired for each individual valve and sets this as the initial valve opening current before agent delivery control takes over during a therapy mode following the liftoff finder routine. This approach allows for faster valve opening without overshoot that may impact agent delivery performance, especially during low agent flow rate targets. The technical benefits include improved response time, more precise control of anesthetic agent delivery, and better handling of valve-to-valve variations without compromising performance.

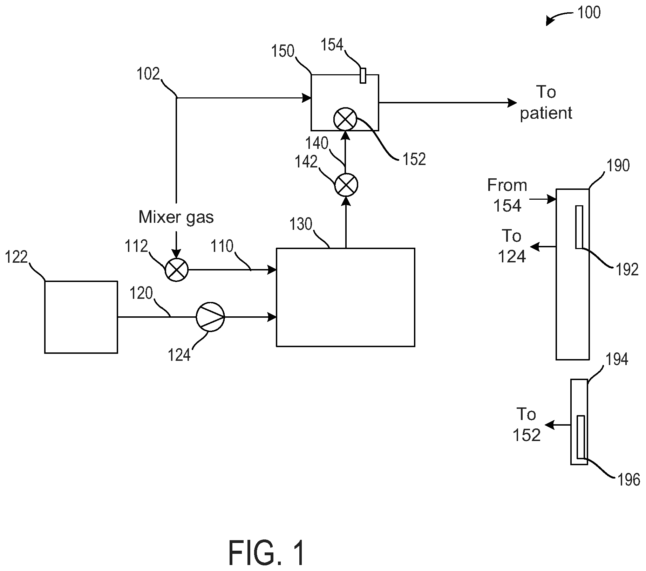

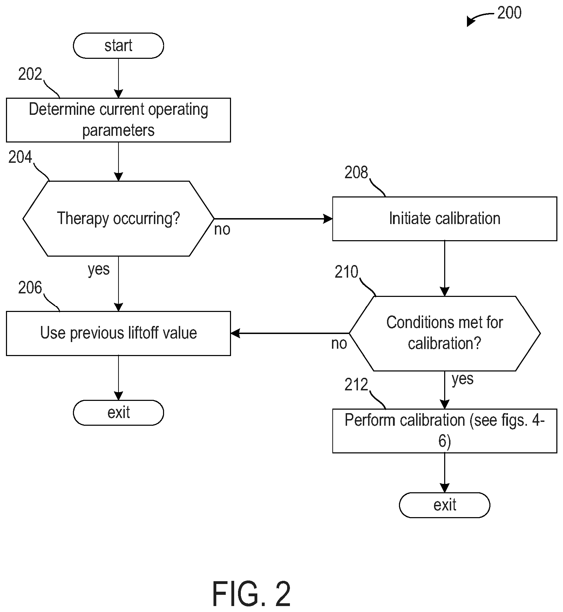

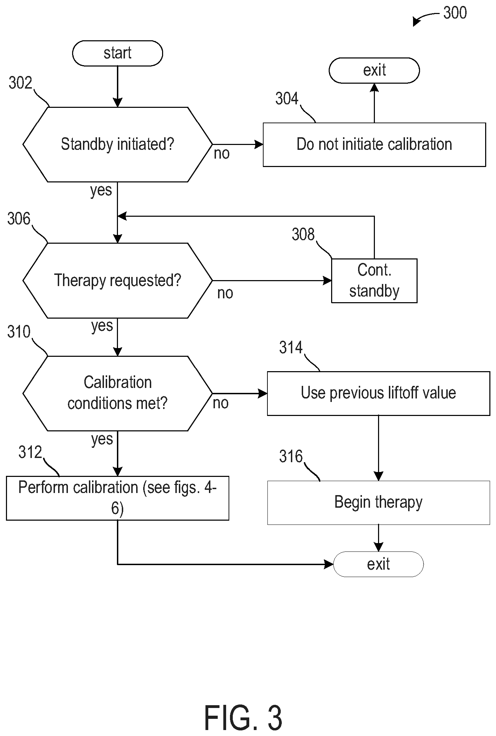

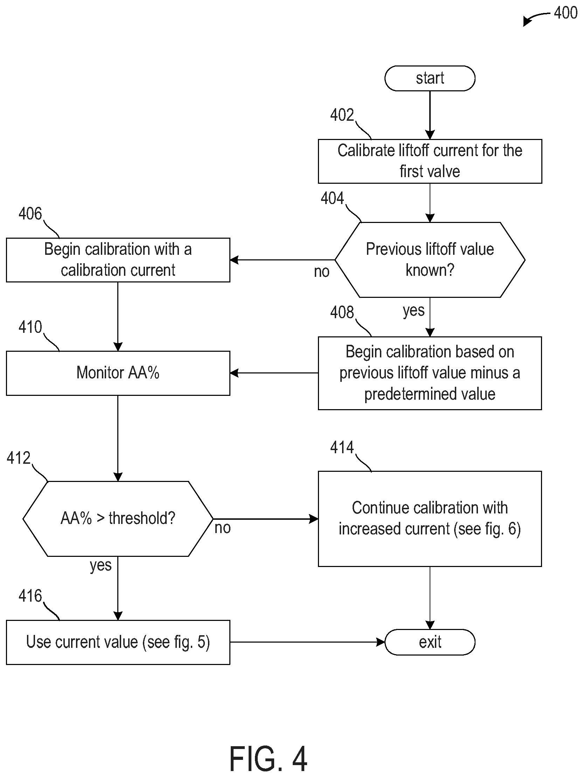

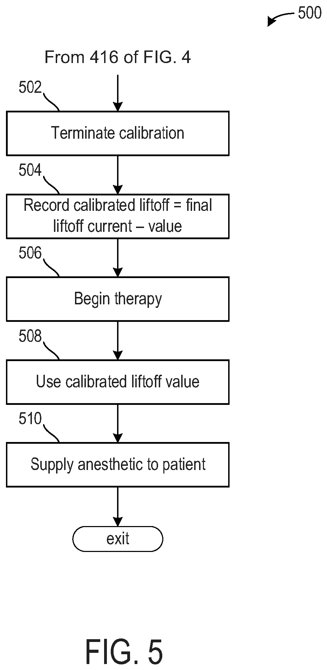

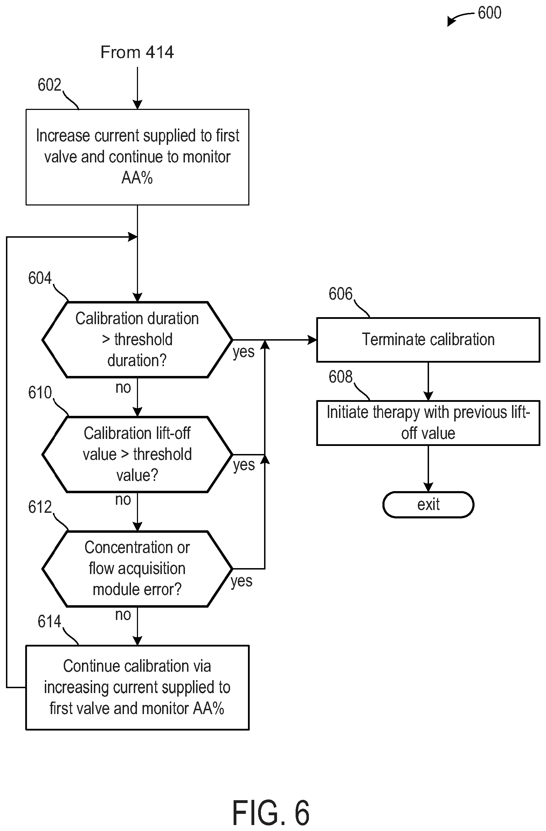

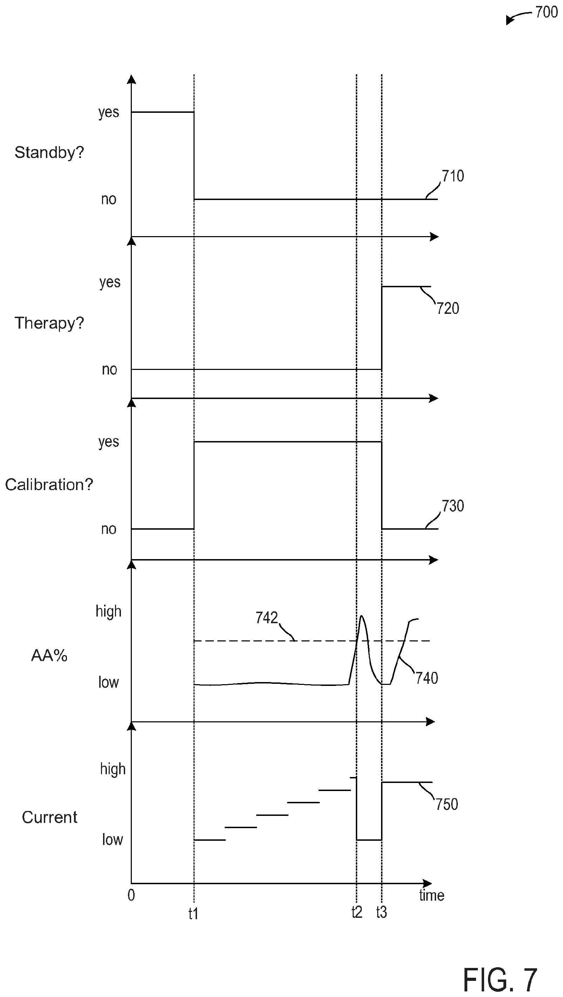

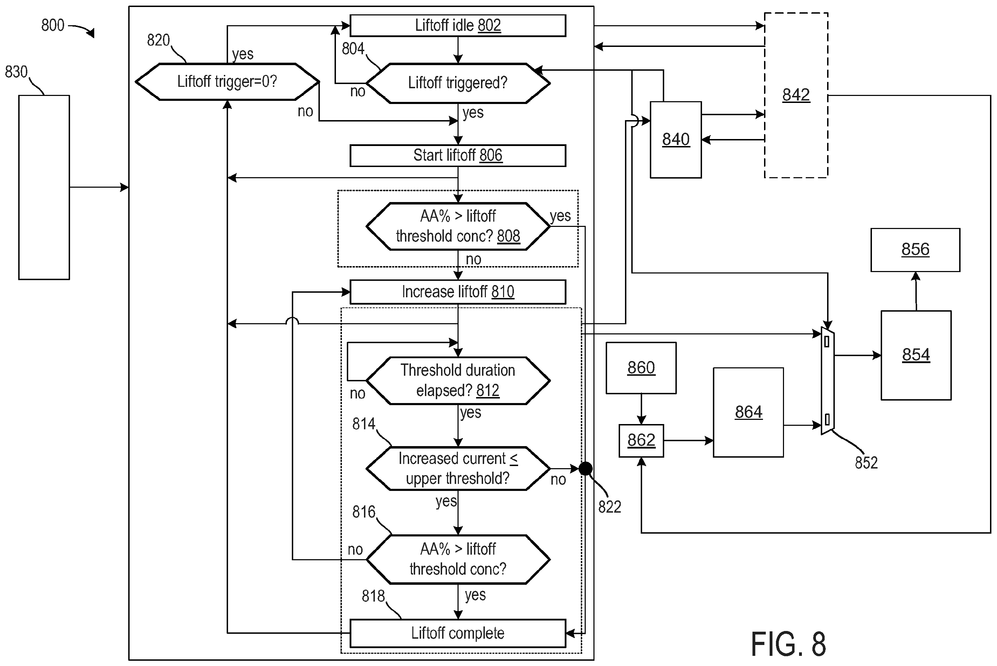

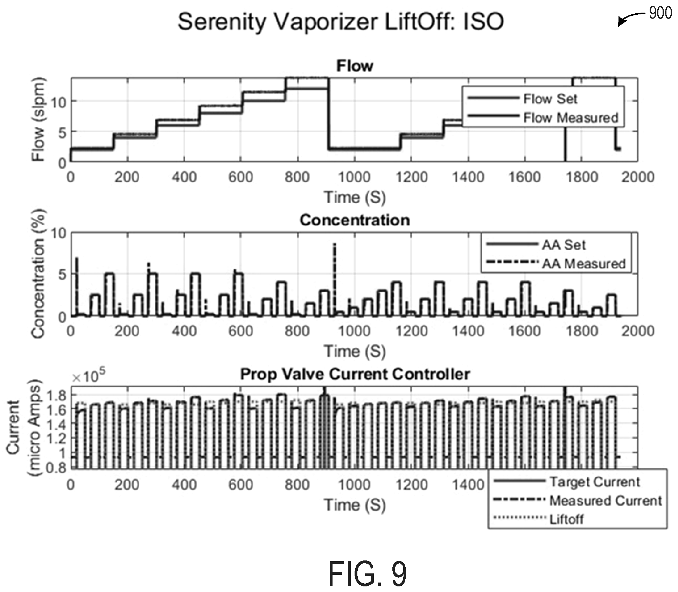

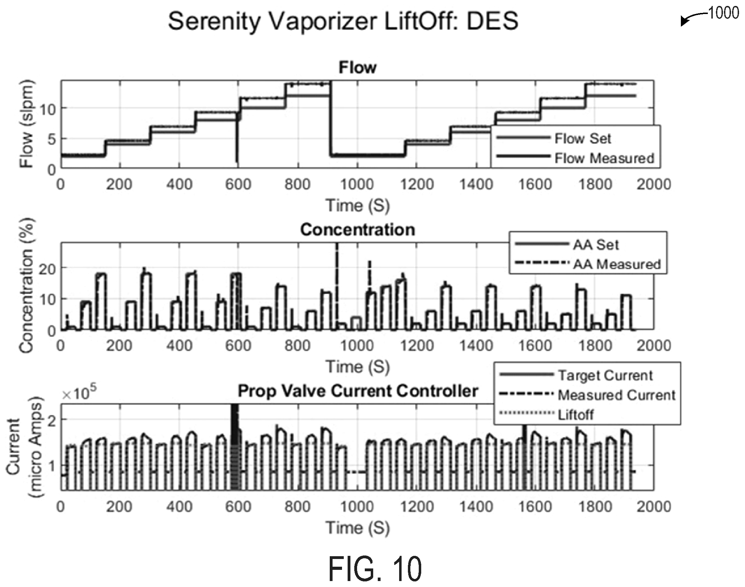

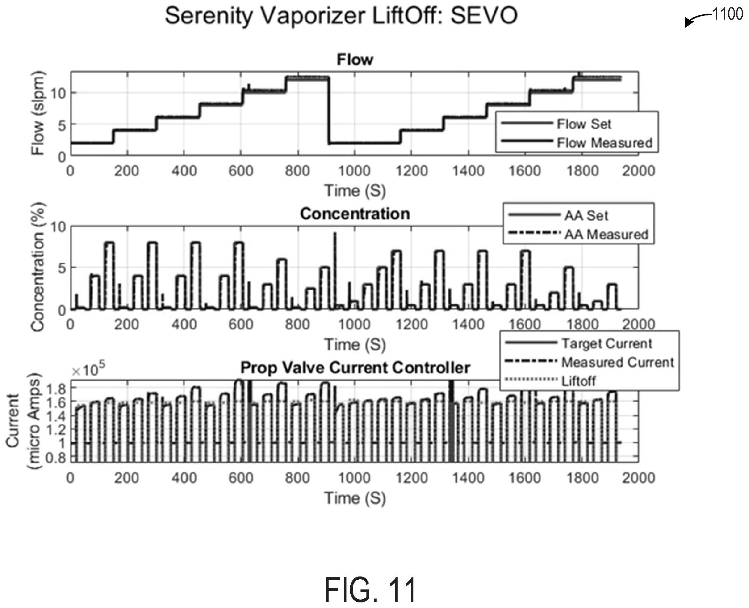

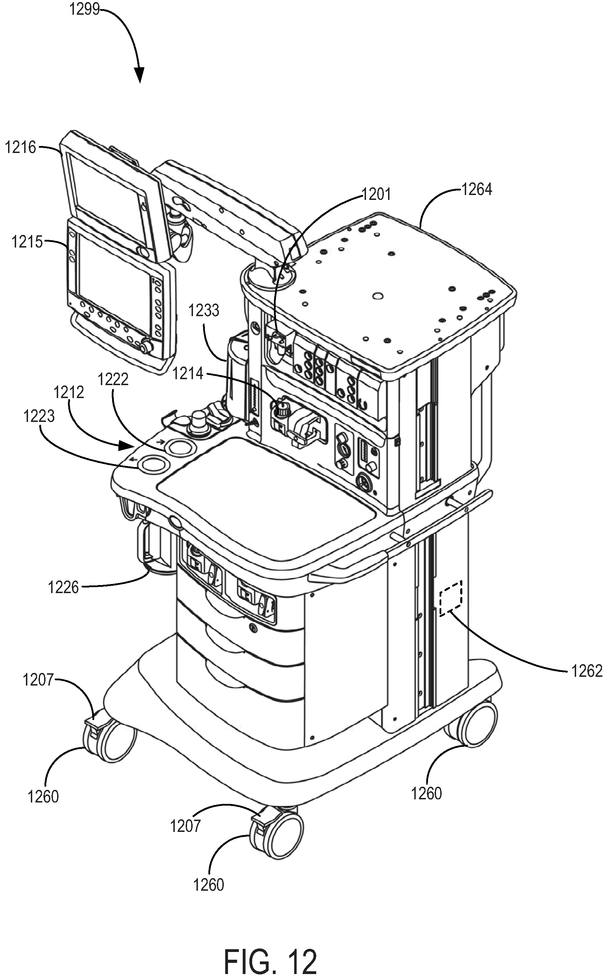

FIG. 1 shows a schematic of a vaporizer. FIG. 2 shows a method for determining if calibration conditions are met. FIG. 3 shows a method for determining a mode of operation of an anesthetic vaporizer. FIG. 4 shows a method for initiating a calibration mode of a valve of the anesthetic vaporizer. FIG. 5 shows a method for transitioning from the calibration mode to a therapy mode. FIG. 6 shows a method for determining if one or more conditions for deactivating the calibration mode is met. FIG. 7 shows an operating sequence of the anesthetic vaporizer. FIG. 8 shows a method tied to hardware and software of the anesthetic vaporizer for executing the calibration mode. FIG. 9 shows graph illustrating a calibration of a liftoff current of a valve of the anesthetic vaporizer based on a first anesthetic agent. FIG. 10 shows a graph illustrating a calibration of a liftoff current of a valve of the anesthetic vaporizer based on a second anesthetic agent. FIG. 11 shows a graph illustrating a calibration of a liftoff current of a valve of the anesthetic vaporizer based on a third anesthetic agent. FIG. 12 shows a schematic of an anesthetic vaporizer.

Turning now to FIG. 1, it shows a schematic of a vaporizer 100. The vaporizer 100 may include a first mixer gas inlet passage 102 coupled to mixing manifold 150. The vaporizer 100 may include a second mixer gas inlet passage 110. In some examples, an inlet valve 112 may be arranged in the mixer gas inlet passage 110. The inlet valve 112 may be able to control a flow of mixer gas through the mixer gas inlet passage 110 to a vaporization chamber 130. The mixer gas may include oxygen, nitrogen, and other elements.

The vaporizer 100 may further include an anesthetic agent passage 120 coupled to each of a sump 122 and the vaporization chamber 130. A pump 124 may be arranged in the anesthetic agent passage 120 and configured to draw anesthetic from the sump 122, through the anesthetic agent passage 120, and into the vaporization chamber 130.

An outlet passage 140 may fluidly couple the vaporization chamber 130 to a mixing manifold 150. An outlet valve 142 may be arranged in the outlet passage 140 and configured to control a flow of vaporized anesthetic agent to the mixing manifold 150. In one example, the outlet valve 142 is a check valve configured to move to only a fully open or a fully closed position.

A proportional valve 152 may be configured to control a flow rate of vaporized anesthetic agent into the mixing manifold 150. The proportional valve 152 may be actuated from a fully closed position to a fully open position or any position therebetween (e.g., greater than 0% open and less than 100% open).

A sensor 154 may be arranged in the mixing manifold 150 and configured to sense an amount of anesthetic. In some examples, an operation of the proportional valve 152 may be based on feedback from the sensor 154.

The vaporizer 100 may further include a controller 190. The controller 190 may include memory 192. Memory 192 may be non-transitory memory including instructions stored thereon that when executed cause the controller to execute one or more functions. For example, the controller 190 may be in communication with the sensor 154.

The vaporizer 100 may further include a controller 194. The controller 194 may include memory 196. Memory 196 may be non-transitory memory including instructions stored thereon that when executed cause the controller to execute one or more functions. During some conditions, the controller 190 may be configured to calibrate an opening duty cycle (e.g., current demand) of the proportional valve 152. In one example, the controller 194 is a first controller and the controller 190 is a second controller. The first controller 194 may be a current controller and the second controller 190 may be a concentration controller.

For example, the vaporizer 100 may be demanded to deliver a range of anesthetic agent flow rates by controlling the proportional valve 152 via operation of the first controller 194 and the second controller 190. During low flow agent control, the proportional valve 152 is controlled to a lower end of a valve opening curve. The proportional valve 152, which is an electromechanical valve in one example, may include a relatively high opening current that can vary due to manufacturing tolerances. The opening current may further change due to valve ageing and environmental conditions. This variance may present a challenge when a fast opening time is desired during low flow conditions where a valve operating range is close to the opening current. Thus, the memory 196 may include instructions that when executed cause the first controller of the controller 194 to calibrate an opening current of the proportional valve 152 to avoid oversupplying anesthetic agent to the mixing manifold 150 while the second controller 190 is unused. The first controller and the second controller may both be used during a therapy mode. The calibration routine is described in greater detail below.

"Systems," "units," "sensors," or "modules" may include or represent hardware and associated instructions (e.g., software stored on a tangible and non-transitory computer readable storage medium, such as a computer hard drive, ROM, RAM, or the like) that perform one or more operations described herein. The hardware may include electronic circuits that include and/or are connected to one or more logic-based devices, such as microprocessors, processors, controllers, or the like. These devices may be off-the-shelf devices that are appropriately programmed or instructed to perform operations described herein from the instructions described above. Additionally or alternatively, one or more of these devices may be hard-wired with logic circuits to perform these operations.

Turning now to FIG. 2, it shows a method 200 for executing a calibration of a valve in an anesthetic vaporizer. Instructions for carrying out method 200 may be executed by one or more controller based on instructions stored on a memory of the controllers and in conjunction with signals received from sensors of the vaporizer system, such as the sensors described above with reference to FIG. 1. The controller may employ actuators of the vaporizer system to adjust operation of the vaporizer, according to the methods described below.

The method 200 begins at 202, which includes determining current operating parameters. Current operating parameters of the vaporizer system may include determining an operating mode such as standby, therapy, calibration, and off.

At 204, the method 200 may include determining if therapy is occurring. Therapy may be occurring if anesthetic is being delivered to a patient or if anesthetic is requested to be delivered to the patient. If therapy is occurring or already initiated, then at 206, the method 200 may include using a previous liftoff value. The previous liftoff value may correspond to a current supplied to a first valve (e.g., the proportional valve 152 of FIG. 1) to actuate the first valve from a closed position to an open position during a previous therapy. The first controller and the second controller may be used during therapy.

If therapy is not occurring or is not initiated, then at 208, the method 200 may include initiating calibration. Initiating calibration may include switching from a standby mode to a calibration mode. During the calibration mode, a liftoff current for the first valve may be determined. The liftoff current is the current used to move the first valve out of a fully closed position. That is to say, when the first valve is moved from the fully closed position to an open position, a liftoff current is used. When the first valve is actuated between different open positions, an actuation current is used. When the first valve is actuated from an open position to a fully closed position, current may no longer be supplied to the first valve.

At 210, the method 200 may include determining if conditions are met for calibration. Calibration conditions may include a threshold flow rate and anesthetic agent concentration. In one example, the threshold flow rate is 2 standard liters per minute (SLPM) and the anesthetic agent concentration is 0.15%. Other threshold flow rates and anesthetic agent concentrations may be used without departing from the scope of the present disclosure. Other conditions for the calibration may include no flags being set related to an error in a concentration acquisition module, a flow acquisition module, a current acquisition module, and a controller configured to provide current to the first valve.

If calibration conditions are not met, then the method 200 may proceed to 206, which includes using a previous liftoff current value as described above.

If calibration conditions are met, then at 212, the method 200 may include performing the calibration. Performing the calibration may include applying a first current to the first valve and monitoring an anesthetic agent percentage. The calibration mode is described in greater detail with respect to FIGS. 4-6.

Turning now to FIG. 3, it shows a method 300 operating a standby mode. The method 300 begins at 302, which includes determining if a standby is initiated or occurring. A standby mode may include where the vaporizer is on but anesthetic is not being provided to the patient. During the standby mode, a liftoff trigger may be equal to 0 and liftoff may be in an idle state. As such, the first valve may be maintained in the closed position and current may not be supplied thereto.

If standby is not initiated or occurring, then at 304, the method 300 may include not initiating a calibration. As such, the first valve may not be supplied current and liftoff may not occur. The anesthetic agent percentage may not be monitored.

If standby is initiated, then the method 300 may proceed to 306, which includes determining if therapy is requested. Therapy may be requested if a medical provider requests to supply anesthetic to a patient. If therapy is not requested, then method 300 may proceed to 308, which includes continuing standby operation. The first valve, the second valve, and the third valve may be maintained closed.

If therapy is requested, then method 300 may proceed to 310, which includes determining if calibration conditions are met. Calibration conditions may include a threshold flow rate and anesthetic agent concentration. In one example, the threshold flow rate is 2 standard liters per minute (SLPM) and the anesthetic agent concentration is 0.15%. Other threshold flow rates and anesthetic agent concentrations may be used without departing from the scope of the present disclosure. Other conditions for the calibration may include no flags being set related to an error in a concentration acquisition module, a flow acquisition module, a current acquisition module, and a controller configured to provide current to the first valve.

If calibration conditions are met, then at 312, the method 300 may include performing the calibration. The calibration is described in greater detail below with respect to FIGS. 4-6.

If calibration conditions are not met, then at 314, the method 300 may include using a previous liftoff current value as described above.

At 316, the method 300 may include beginning therapy. The previous liftoff current value may be supplied to the first valve. Following a determined amount of time, a current value corresponding to a desired open position of the first valve may be supplied to the first valve, wherein the open position current value may be different than the previous liftoff current value. In one example, the open position current value may be less than the previous liftoff current value. In another example, the open position current value may be greater than the previous liftoff current value.

Turning now to FIG. 4, it shows a method 400 for calibrating the first valve. The method 400 begins at 402, which includes calibrating a liftoff current for the first valve during a calibrating mode. Calibrating the liftoff current may occur outside of the therapy mode. In one example, only the first controller may be used during the calibration. In one example, the first controller operates to supply current to the first valve. The second controller may be unused (e.g., inactive). The second controller may be configured to receive feedback regarding an anesthetic concentration and adjust operating parameters based on the anesthetic concentration. Additionally or alternatively, medical gas may be blocked from flowing to the mixing chamber during the calibration. As will be described herein, the calibration is executed outside of a therapy session such that anesthetic does not flow to a patient despite the calibration being executed following a therapy request. The calibration may be executed within a threshold duration such that the opening of the first valve occurs within a determined timespan to minimize delays. In one example, the calibration occurs within an amount of time less than a time needed to open the first valve outside of the calibrations as will be described in greater detail below.

At 404, the method 400 may include determining if a previous liftoff value is known. The previous liftoff value being a current supplied during a previous therapy session to actuate the first valve out of a fully closed position to a desired open position. If the previous liftoff value is not known, then at 406, the method 400 may include beginning the calibration with a starting calibration current. In one example, the starting calibration current is based on a value at a lower end of an opening current range. For example, if the opening current range is 120 to 180 milliampere (mA), then the calibration may begin at 120 mA. The first controller may supply the current to the first valve.

If the previous liftoff value is known, then at 408, the method 400 beginning the calibration based on a previous liftoff value minus a predetermined value. In one example, the predetermined value may be equal to a step value of the calibration. For example, the current supplied to the first valve may be adjusted by a fixed value equal to the step value. In this way, the value of the previous liftoff value may be reached during a second step of the calibration, in one example. Additionally or alternatively, the predetermined value may be equal to a percentage of the step value.

At 410, following 406 or 408, the method 400 may include monitoring the anesthetic agent percentage. The anesthetic agent percentage may be sensed via a sensor arranged in a mixing manifold (e.g., sensor 154 of FIG. 1) or via a sensor coupled to the outlet passage (e.g., the outlet passage 140 of FIG. 1). In one example, the anesthetic agent percentage may be monitored for a determined amount of time following supplying the opening current to the first valve.

At 412, the method 400 may include determining if the anesthetic agent percentage is greater than a threshold anesthetic agent percentage. The threshold anesthetic agent percentage may be based on an estimated anesthetic agent percentage of a lowest flow rate of the first valve. The threshold anesthetic agent percentage may be based on a non-zero, positive number. In another example, the threshold anesthetic agent percentage may be a calibration threshold percentage.

If the anesthetic agent percentage is not greater than the threshold anesthetic agent percentage, then at 414, the method 400 may include continuing the calibration with an increased current supplied to the first valve. The current may be increased relative to the starting calibration current or the previous liftoff value minus the increasing a current supplied to a first valve and continuing to monitor the anesthetic agent percentage. Increasing the current supplied may include adjusting the current by a fixed value such as a fixed current value or a fixed percentage. The fixed current value may be used in a stepwise calibration of the first valve. The fixed percentage may result in larger increases of the current applied as the calibration progresses. For example, 10% of an original current applied to the first valve is less than 10% of a third current applied to the first valve. The continuation of the calibration is described in greater detail below with respect to FIG. 6.

If the anesthetic agent percentage is greater than the threshold anesthetic agent percentage, then the method 400 proceeds to 416, which include using the calibrated current value. The calibrated current value may be associated with a current value supplied to the first valve directly prior to the anesthetic agent percentage greater than the threshold anesthetic agent percentage being sensed. Using and processing the calibrated current value is described in greater detail with respect to FIG. 5.

Turning now to FIG. 5, it shows a method 500 for transitioning from the calibration mode to a therapy mode. The method 500 begins at 502, following 416 of FIG. 4, which include terminating the calibration. Terminating the calibration may include returning the first valve to a closed position. Additionally or alternatively, terminating the calibration may include maintaining the first valve in the open position and no longer adjusting a current supplied to the first valve based on the calibration process.

At 504, the method 500 may include recording a calibrated liftoff current, which is equal to a final liftoff current applied to the valve minus a value. In one example, the value may be based on a delay of the anesthetic agent sensor. In one example, the calibrated liftoff current is between a second to last liftoff current applied to the first valve and the final liftoff current applied to the valve. That is to say, the calibrated liftoff current is greater than the second to last liftoff current and less than the final liftoff current used during the calibration mode.

At 506, the method 500 may include beginning therapy. Therapy may include mixing anesthetic agent with a mixing gas and supplying the anesthetic to a patient.

At 508, the method 500 may include using the calibrated liftoff value. As such, a current equal to the calibrated liftoff value is applied to the first valve.

At 510, the method 500 may include supplying anesthetic to the patient. Additionally or alternatively, one or more of the second valve and the third valve may be opened.

Turning now to FIG. 6, it shows a method 600 for continuing the calibration and determining if conditions for the calibration mode are no longer met. Proceeding from 414 of FIG. 4, the method 600 begins at 602, which includes increasing a current supplied to the first valve and continuing to monitor the anesthetic agent percentage. An amount of time may elapse between supplying the initial current (e.g., a first current such as the starting calibration current or the previously known liftoff value) to the first valve and a second current to the first valve. In one example, no current may be supplied to the first valve during the amount of time.

Following increasing the current supplied to the first valve and continuing to monitor the anesthetic agent percentage, the method 600 proceeds to 604. At 604, the method 600 may include determining if a calibration duration is greater than a threshold calibration duration. The threshold calibration duration may be based on a non-zero, positive number. In one example, the threshold calibration duration is based on an opening time of the first valve outside of the calibration. For example, the threshold calibration duration may be based on an opening time of the valve when executing the therapy mode directly following the standby mode (e.g., without a calibration mode occurring between the therapy mode and the standby mode). In this way, the calibration mode may be expected to determine a calibrated liftoff current in an amount of time equal to or less than a time used to open the first valve without the calibration mode. By doing this, anesthetic delivery to the patient may not be delayed due to the calibration. Additionally or alternatively, the threshold duration may be based on a time in which anesthetic delivery to the patient is demanded.

If the calibration duration has exceeded the threshold duration, then at 606, the method 600 may include terminating the calibration.

At 608, the method 600 may include initiating therapy with the previous lift-off value. Therapy may further include opening the second and/or third valves. In one example, the second controller may be activated such that each of the first controller and the second controller may be used during the therapy mode.

If the calibration duration does not exceed the threshold duration (e.g., is less than or equal to), then at 610, the method 600 may include determining if a calibration current value is greater than a threshold upper current value. In one example, the threshold upper current value is based on a non-zero, positive value. The threshold upper current value may be equal to an upper limit of a current range in which the first valve is known to be opened. For example, if the first valve opens in response to a current between 120 - 180 mA, which may be a range set by a manufacturer or determined over time based on an operation of the first valve, then the threshold upper current value may be equal to 180 mA. If the calibration mode includes applying a current value that is greater than the threshold upper current value, then the method 600 may proceed to 606 as described above and terminate the calibration mode and transition to therapy mode.

If the calibration includes applying a current value that does not exceed the threshold upper current value, then at 612, the method 600 may include determining if a concentration or flow acquisition module error is present. The concentration or flow acquisition module error may be related to a degradation of the sensor or other component of the vaporizer. If the concentration and/or flow acquisition module error is present, then the method 600 proceeds to 606 as described above and terminates the calibration mode and initiates the therapy mode.

In one example, the methods of FIG. 2-6 illustrate an example operation of the vaporizer including a calibration of a liftoff current of a proportional valve of the vaporizer. The calibration is executed immediately after a standby mode and immediately prior to a therapy mode when calibration conditions are met. In one example, the therapy mode is executed immediately following the calibration. In some examples, the calibration is executed only in response to a therapy mode being requested while the vaporizer is in the standby mode.

Turning now to FIG. 7, it shows an operating sequence 700 of the anesthetic vaporizer. Plot 710 illustrates if standby is occurring. Plot 720 illustrates if therapy is occurring. Plot 730 illustrates if calibration is occurring. Plot 740 illustrates an anesthetic agent percentage and dashed line 742 indicates a threshold anesthetic agent percentage. Plot 750 illustrates a current supplied to the first valve. Time increases from a left to a right side of the figure.

Prior to t1, the standby mode is occurring. Thus, the therapy mode and the calibration mode are not occurring. As such, therapy and calibration are not occurring. The anesthetic agent percentage is not sensed and there is no current applied to the first valve as a liftoff trigger is equal to zero.

At t1, standby is no longer occurring and calibration is initiated. A first amount of current is applied to the first valve. The first amount of current may be equal to a starting calibration current or a previous liftoff current used during a previous therapy mode. Between t1 and t2, the current applied to the first valve increases in a stepwise manner. In one example, between each iteration of current provided to the first valve, current supply to the first valve may be stopped. In this way, the first valve may be closed between iteration in which current is supplied.

At t2, the anesthetic agent percentage is greater than the threshold anesthetic agent percentage. As such, the current supplied to the first valve at t2 is the calibrated liftoff current value. A value may be subtracted from the calibrated liftoff current value to generate a corrected value to account for a delay of the sensor configured to sense the anesthetic agent percentage. Between t2 and t3, the first valve no longer receives current and the anesthetic agent percentage is decreased to zero. The corrected value may be saved to memory.

At t3, therapy is initiated. The corrected value of the calibrated liftoff current value is applied to the first valve. After t3, the anesthetic agent percentage increases and anesthetic may be supplied to the patient.

Turning now to FIG. 8, it shows a method 800 illustrated with hardware configured to execute and/or implement different portions of the method 800. For example, feedback from a calibration acquisition module 830 may be used by the method 800 to compare the anesthetic agent percentage to the liftoff threshold anesthetic percentage. In one example, the calibration acquisition module 830 may represent software of a computing processor of the anesthetic vaporizer.

The method 800 begins at 802, which includes where liftoff is idle. Liftoff may be idle if liftoff is not triggered (e.g., not requested). Liftoff may not be requested during the standby mode. When liftoff is not triggered, the first valve may be moved to a liftoff idle state, which may be associated with a fully closed position. When in the fully closed position, anesthetic agent may be blocked from flowing from the vaporization chamber to the mixing chamber.

At 804, the method 800 may include determining if liftoff is triggered. Liftoff may be triggered in response to therapy being requested. As such, a liftoff trigger value may switch from zero at 802 to 1 at 804 in response to therapy being requested. In one example, calibration is initiated only in response to therapy being requested. In this way, the anesthetic vaporizer does not flow anesthetic to the mixing chamber during conditions outside of the therapy mode. Said another way, the calibration may not be initiated independently of the therapy mode and is only initiated following the standby mode and prior to the therapy mode.

A therapy state controller 840 may be a design module configured to control operations in programmable logic. The therapy state controller 840 may trigger liftoff based on a command from a processor 842 and record the completion of the liftoff for the processor 842. Processor 842 may track if a liftoff is triggered at 804 with a current time. In this way, a time elapsed since the liftoff trigger may be tracked.

If liftoff is not triggered, then the method 800 returns to 802 and maintains liftoff at idle.

If liftoff is triggered, then the method 800 may proceed to 806, which includes starting liftoff. Starting liftoff may include supplying an amount of current to the first valve. The amount of current may be based on a previous liftoff current used during a previous therapy mode. Alternatively, the amount of current may be based on a starting calibration current, which may be equal to a lower value of a calibration current range in which the first valve is known to open.

At 808, the method 800 may include determining if an anesthetic agent percentage is greater than a liftoff threshold anesthetic concentration. The liftoff threshold anesthetic concentration may be based on a non-zero, positive number. Additionally or alternatively, the liftoff threshold anesthetic concentration may be proportional to the amount of current supplied to the first valve. As the amount of current increases, the liftoff threshold anesthetic concentration may also increase, in one example. The anesthetic agent percentage may be determined based on feedback from a sensor, such as anesthetic sensor 154 arranged in the mixing manifold 150 of FIG. 1.

If the anesthetic agent percentage is greater than the liftoff threshold anesthetic concentration, then a liftoff current value used when the anesthetic agent percentage exceeds the liftoff threshold anesthetic concentration is used and a liftoff flag is set. In one example, the liftoff current is unchanged. That is to say, a calibrated liftoff current is equal to the previously used liftoff current. As such, during a therapy mode following the calibration mode, a target current provided to the first valve for liftoff is equal to the previously used liftoff current and the first liftoff current used during the calibration mode.

If the anesthetic agent percentage is not greater than the liftoff threshold anesthetic concentration, then at 810, the method 800 may include increasing a liftoff current. Increasing the liftoff current may include increasing the current by a fixed amount, a percentage, or other amount.

At 812, the method 800 may include determining if a threshold liftoff duration has elapsed. The threshold liftoff duration may be based on a non-zero, positive number. The threshold liftoff duration may be based on a time used to one or more of open the first valve and flow anesthetic from the vaporization chamber to the mixing chamber. In one example, the threshold liftoff duration is a wait time between the step increment of the liftoff current and is based on a response time of the anesthetic agent sensor.

If the threshold liftoff duration has not elapsed, then the method 800 continues to monitor the threshold liftoff duration.

Once the threshold liftoff duration has elapsed, then the method 800 may proceed to 814, which includes determining if the increased liftoff current is less than or equal to an upper threshold calibration range current. The upper threshold calibration range current may be based on the known opening current range of the first valve, which may be set by a manufacturer or determined during an initial calibration of the first valve. If the increased liftoff current is not less than or equal to the upper threshold calibration range current and as such the increased liftoff current is greater than the upper threshold calibration range current, then the method 800 may proceed to 822. The calibration mode may be terminated at 822 and a previous liftoff value may be used in a subsequent therapy mode. In one example, when logic gate is activated at 822, a calibrated current value is not used during the subsequent therapy mode.

If the increased liftoff current is less than or equal to the upper threshold calibration range current, then method 800 may proceed to 816, which includes determining if an anesthetic agent percentage is greater than a liftoff threshold anesthetic concentration, similar to 808 as described above. If the anesthetic agent percentage is not greater than the liftoff threshold anesthetic concentration, and as such the anesthetic agent percentage is less than the liftoff threshold anesthetic concentration, then the method 800 may return to 810 to increase the liftoff current. The calibration mode may continue to increase the liftoff current until one or more of the anesthetic agent percentage is greater than the liftoff threshold anesthetic concentration, the liftoff current used during the calibration is not less than or equal to the upper threshold calibration range current, a total calibration duration exceeds a threshold total calibration duration, and a concentration acquisition module degradation is present.

The method 800 may proceed to 818 following 822 or following determination that the anesthetic agent percentage is greater than the liftoff threshold anesthetic concentration. At 818, the method 800 may include liftoff being complete. When liftoff is complete, the first valve may be moved from an open position, where fluid may flow from the vaporization manifold to the mixing manifold, to a closed position, which may block fluid flow from the vaporization manifold to the mixing manifold. In one example, when liftoff is complete, the final current supplied to the first valve during the calibration mode may be recorded and processed as a target calibration current to be used during a subsequent therapy mode liftoff start.

The method 800 may proceed to 820 following 818, which includes determining if a liftoff trigger is equal to 0. If the liftoff trigger is equal to 0, then liftoff may not be requested and the method 800 may return to 802 where liftoff is idle and the first valve is closed. If the liftoff trigger is not equal to 0, then liftoff may be requested and the method 800 may proceed to 806 to start liftoff.

Steps 812, 814, 816, 818, and 822 may be tracked via the processor 842. The therapy state controller 840 may be in communication with a processor 842. The processor 842 may include non-transitory instructions that when executed enable the processor 842 to execute the method 800 and provide signals to programmable logic of the system. In one example, the processor 842 is a central processor that may receive liftoff values used and calibrated in the method 800, provide a liftoff starting current to the method 800, and modify a subsequent therapy mode based on results of the method 800. The processor 842 may be in communication with the therapy state controller 840 and the calibration acquisition module.

The multiplexer 852 may be configured to receive two or more inputs and provide an output based on the inputs received. For example, the multiplexer 852 may receive inputs related to a present calibration current being used, a liftoff trigger value from the therapy state controller 840, and current controller target from a second controller 864. The multiplexer 852 may output a current controller target based on the inputs received via executing the calibration mode to a first controller 854. The first controller 854 may output to a proportional valve 856. The proportional valve 856 is arranged between a vaporization chamber and a mixing manifold of an anesthetic vaporizer. The processor 842 outputs computed liftoff to an adder module 862. The adder module 862 adds the feedforward value from feed forward scheduler 860 and computed liftoff from processor 842 and provides the output to second controller 864.

In one example, each of the therapy state controller 840, multiplexer 852, the first controller 854, the feedforward scheduler 860, the adder module 862, the second controller 864, and the calibration acquisition module 830 represent a system or module of the processor 842 including programmable logic. In one example, the second controller 864 is bypassed during the liftoff routine and may be used in the therapy mode to provide the target current for the first controller 854. In one example, the first controller 854 and the second controller 864 are two controllers in cascade. As such, an output of one controller (e.g., the first controller 854) is used as a setpoint of the other controller (e.g., the second controller 864). The second controller 864 may be identical to controller 190 of FIG. 1 and first controller 854 may be identical to the controller 194 of FIG. 1.

As used herein, the terms "system" or "module" may include a hardware and/or software system that operates to perform one or more functions. For example, a module or system may include a computer processor, controller, or other logic-based device that performs operations based on instructions stored on a tangible and non-transitory computer readable storage medium, such as a computer memory. Alternatively, a module or system may include a hard-wired device that performs operations based on hard-wired logic of the device. Various modules or systems shown in the attached figures may represent the hardware that operates based on software or hardwired instructions, the software that directs hardware to perform the operations, or a combination thereof. For example, the therapy state controller 840, multiplexer 852, the first controller 854, the feedforward scheduler 860, the feedforward module 862, the second controller 864, and the calibration acquisition module 830 may be implemented using hardware, software, firmware, or a combination thereof. In some aspects, several portions of the subject matter described herein may be implemented via Application Specific Integrated Circuits (ASICs), Field Programmable Gate Arrays (FPGAs), semiconductor devices based around a matrix of configurable logic blocks (CLBs), connected via programmable interconnects. Some aspects of the embodiments disclosed herein, in whole or in part, can be equivalently implemented in standard integrated circuits, as one or more computer programs running on one or more computers (e.g., as one or more programs running on one or more computer systems), as one or more programs running on one or more processors (e.g., as one or more programs running on one or more microprocessors), as firmware, or as a combination thereof.

Turning now to FIGS. 9, 10, and 11, they show calibration results of the calibration acquisition module (e.g., calibration acquisition module 830 of FIG. 8) with different anesthetic agent types. Calibration result 900 of FIG. 9 was implemented and tested on Isoflurane (ISO). Calibration result 1000 of FIG. 10 was implemented and tested on Desflurane (DES). Calibration result 1100 of FIG. 11 was implemented and tested on Sevoflurane (SEVO). Each of the calibration results 900, 1000, and 1100 plots flow in SLPM against time, an anesthetic agent percentage against time, and a current in mA against time. Time increases from a left to a right side of the figures.

Turning now to FIG. 12, it shows an example an anesthesia machine 1299, which is a non-limiting example of a patient therapy device, such as the vaporizer 100 of FIG. 1. Anesthesia machine 1299 includes a frame 1264 supported by casters 1260, where the movement of the casters may be controlled (e.g., stopped) by one or more locks 1207. In some examples, the frame 1264 may be formed of a plastic material (e.g., polypropylene). In other examples, the frame 1264 may be formed from a different type of material (e.g., metal, such as steel).

Anesthesia machine 1299 also includes respiratory gas module 1201, ventilator 1212, vaporizer 1214, anesthesia display device 1215, and patient monitoring display device 1216.

Vaporizer 1214 may be in the form of one or more removable cassettes such that a supply of anesthetic agent can be removed from the cassette and such that different types of anesthetic agents can be supplied to the anesthesia machine by simply removing the cassette and replacing it with a different cassette for a different anesthetic agent. When more than one anesthetic agent is to be delivered, more than one cassette may be included with a different anesthetic agent in each cassette. The vaporizer 1214 includes a housing having a drug reservoir that contains a supply of anesthetic agent to be delivered to a patient. The drug reservoir may have a discharge opening formed in a back wall of the housing that is configured to receive a discharge tube (not shown), which is part of the anesthesia machine, forming a gas-tight seal for delivery of anesthetic vapor to a patient.

Vaporizer 1214 may include at least one absorbent wick within the reservoir. A passageway allows fresh gas from a gas source to pass to the absorbent wick(s). Vaporized liquid from the absorbent wick(s) and accompanying fresh gas may flow to the back of the drug reservoir and out the discharge opening in the housing. A lower portion of the drug reservoir may contain the liquid anesthetic agent and an upper portion of the reservoir may contain the vaporized anesthetic agent and breathing gases. During operation, a combination of temperature and pressure affect the liquid anesthetic agent and cause it to vaporize into the breathing gases. The gases carrying the vaporized agent are then discharged through the discharge opening.

A rear of the anesthesia machine 1299 may include one or more pipeline connections to facilitate coupling of the anesthesia machine to pipeline gas sources. Additionally, the rear of the anesthesia machine may include a cylinder yoke, via which one or more gas-holding cylinders may be coupled to the anesthesia machine. Thus, through the pipeline connection and/or cylinder connections, gas may be provided to the anesthesia machine, where the gas may include but is not limited to fresh gas, oxygen, and nitrous oxide. The gas that enters the anesthesia machine may mix with the vaporized anesthetic agent at the vaporizer, as described above, and be supplied to a patient via the ventilator. In some embodiments, the rear of the anesthesia machine may also include a serial port, a collection bottle connection, cylinder wrench storage, anesthesia gas scavenging system, main power inlet, system circuit breaker, equipotential stud, outlet circuit breaker, and isolated electrical outlet.

The ventilator 1212 may include an expiratory check valve 1222, inspiratory check valve 1223, absorber canister 1226, and bellows assembly 1233. When a patient breathing circuit is coupled to the ventilator, the breathing gases (e.g., fresh gas, oxygen, and/or nitrous oxide mixed with vaporized anesthetic agent) exit the machine from an inspiratory port (which may be positioned at the same location as the inspiratory check valve 1223) and travel to a patient. Expiratory gases from the patient re-enter the anesthesia machine via an expiratory port (which may be positioned at the same location as the expiratory check valve 1222), where the carbon dioxide may be removed from the expiratory gases via the absorber canister 1226.

During operation of the vaporizer 1214, an operator (e.g., an anesthesiologist) may adjust an amount of vaporized anesthetic agent that is supplied to a patient by adjusting a flow rate of gases from the gas source(s) (e.g., the gas pipelines) to the vaporizer. The flow rate of the gases from the gas source to the vaporizer may be adjusted by the operator via adjustment of one or more flow adjustment devices. For example, the flow adjustment devices may include analog and/or digital adjustment dials and/or other user input devices configured to actuate one or more flow control valves of the anesthesia machine 1299. In one example, a first flow control valve (e.g., first valve 152 of FIG. 1) may be positioned between the gas source(s) and the vaporizer 1214 and may be actuatable via the flow adjustment devices to a fully opened position, a fully closed position, and a plurality of positions between the fully opened position and the fully closed position.

The anesthesia machine may additionally include one or more bypass valves configured to bypass gases from the gas source(s) around the vaporizer 1214. The bypass valves may enable a first portion of gases flowing from the gas source to flow directly from the gas source to the inspiratory port, and a second portion of gases flowing from the gas source may flow through the vaporizer 1214 to mix with the vaporized anesthetic agents prior to flowing to the inspiratory port. By adjusting a ratio of gases flowing to the port via the bypass valves relative to gases flowing to the port via the vaporizer 1214, the operator may control a concentration of vaporized anesthetic agent in gases at the port.

Further, the adjustments described above may be facilitated at least in part based on output from the respiratory gas module 1201. Respiratory gas module 1201 may be configured to measure various parameters of the gases exiting the vaporizer and/or being provided to the patient. For example, respiratory gas module 1201 may measure the concentrations of carbon dioxide, nitrous oxide, and anesthesia provided to the patient. Further, respiratory gas module 1201 may measure respiration rate, minimum alveolar concentration, patient oxygen, and/or other parameters. The output from the respiratory gas module 1201 may be displayed via a graphical user interface displayed on a display device (e.g., device 1215 and/or 1216) and/or used by the controller to provide closed-loop feedback control of the amount of anesthesia provided to the patient.

Ventilator 1212 may optionally be coupled to a breathing circuit (not shown) including a plurality of tubes (e.g., gas passages). The breathing circuit may be coupled between an airway of a patient (e.g., via a breathing mask positioned to enclose the mouth and/or nose of the patient) and the inspiratory port. Gases (e.g., oxygen, or a mixture of oxygen and vaporized anesthetic agents from vaporizer 1214) may flow from the port, through the breathing circuit, and into the airway of the patient, where the gases are absorbed by the lungs of the patient. By adjusting the concentration of vaporized anesthetic agent in the gases as described above, the operator may adjust an amount by which the patient is anesthetized.

During conditions in which the breathing circuit is coupled to the airway, anesthetic agent and/or fresh gases may flow into the airway (e.g., be inhaled by the patient) via the inspiratory check valve 1223. For example, inspiratory check valve 1223 may open automatically (e.g., without input or adjustment by the operator) in response to inhalation of the patient, and may close automatically in response to exhalation of the patient. Similarly, the expiratory check valve 1222 may open automatically in response to exhalation of the patient, and may close automatically in response to inhalation of the patient.

In some examples, the operator may alternately and/or additionally control one or more operating parameters of the anesthesia machine 1299 via an electronic controller 1262 of the anesthesia machine 1299 (shown schematically by FIG. 12). Controller 1262 includes a processor operatively connected to a memory. The memory may be a non-transitory computer-readable medium and may be configured to store computer executable code (e.g., instructions) to be processed by the processor in order to execute one or more routines. The memory may also be configured to store data received by the processor. Controller 1262 may be communicatively (e.g., wired or wirelessly) coupled to one or more external or remote computing devices, such as computing system 120, and may be configured to send and receive various information, such as measured patient monitoring parameters (e.g., the output from the respiratory gas module 1201), electronic medical record information, procedure information, and so forth.

The controller receives signals from the various sensors of the anesthesia machine 1299 and employs the various actuators of the anesthesia machine 1299 to adjust operation of the anesthesia machine 1299 based on the received signals and instructions stored on the memory of the controller. For example, the flow of gases to the inspiratory port may be controlled via an input device (e.g., keyboard, touchscreen, etc.) coupled to the electronic controller of the anesthesia machine 1299. The controller may be electrically coupled to display device 1215 and/or 1216 in order to display operating parameters of the anesthesia machine 1299. The controller may receive signals (e.g., electrical signals) via the input device and may adjust operating parameters of the anesthesia machine 1299 in response (e.g., responsive) to the received signals. For example, the operator may input a desired flow rate of gases (e.g., oxygen) flowing from the gas source to the patient and/or vaporizer 1214.

A corresponding valve position of one or more valves of the anesthesia machine (e.g., a position of one or more bypass valves, as described above) may be empirically determined and stored in a predetermined lookup table or function on the controller. For example, the controller may receive the desired flow rate of gases via the input device and may determine an amount of opening of the one or more bypass valves corresponding to the desired flow rate based on the lookup table, with an input being the desired flow rate and an output being the valve position. The controller may transmit an electrical signal to an actuator of the valve in order to adjust the valve position. In some examples, the controller may compare the desired flow rate of gases to a measured flow rate of gases as measured by a flow rate sensor (e.g., an inspiratory flow sensor).

Controller 1262 is shown in FIG. 12 for illustrative purposes, and it is to be understood that controller 1262 may be located internally of anesthesia machine 1299 and thus may not be visible externally on anesthesia machine. Controller 1262 may include multiple devices/modules that may be distributed across anesthesia machine 1299. In one example, the controller 1262 may be identical to processor 842 of FIG. 8, wherein the controller 1262 is a primary controller with instructions stored in memory thereof that enable the controller 1262 to execute programmable logic in association with one or more sub-controllers/processors.

The technical effect of calibrating an opening current of a valve for an anesthetic vaporizer is to calibrate an opening current of the valve across different models of the anesthetic vaporizer. Additionally or alternatively, the calibration may account for age and location of the valve, which may provide improved operation of the valve.

The lift-off finder routine is used to detect the proportional valve opening current to improve agent delivery response time. The routine is executed when the system enters the therapy mode from standby state and conditions are met. The lift-off start current is set as the previous lift-off value computed minus 10mA, or a default value of 120mA if no previous value is present. This 10mA offset serves two purposes: to gradually open the valve near the lift-off point and to capture any downward shifts in valve lift-off. Once the routine is completed and agent delivery is about to start, the system computes the final lift-off value by subtracting 6mA from the detected lift-off current during the calibration to account for sensor delay and step variation. The system stores the last computed lift-off value for use in the agent delivery controller in case the lift-off finder routine is not executed to completion or produces an error. The lift-off value may be limited to a range of 120-180mA. Conditions for aborting the calibration mode in order to avoid a delayed opening of the valve include timeout conditions if the routine does not converge within the specified time, maximum current clamp limits, and monitoring of concentration/flow acquisition module flags or errors. In these safety scenarios, the system reverts to using the previous known lift-off value or the default 120mA value. Testing across different anesthetic agents (DES, ISO, and SEVO) demonstrated successful calibration and improved response times across various flow rates and target concentrations.

The disclosure also provides support for a method including calibrating a liftoff current of a proportional valve arranged between a vaporization chamber and a mixing manifold of an anesthetic vaporizer via a controller following a standby mode and prior to a therapy mode, wherein the liftoff current is a current supplied to actuate the proportional valve from a closed position to an open position. In a first example of the method, the controller is a first controller, further comprising a second controller that is unused during the calibrating. In a second example of the method, optionally including the first example, the first controller and the second controller are used during the therapy mode. In a third example of the method, optionally including one or both of the first and second examples, the calibrating occurs only in response to the therapy mode being requested during the standby mode. In a fourth example of the method, optionally including one or more or each of the first through third examples, calibrating the liftoff current further comprises initially supplying a previous liftoff current minus a determined value to the proportional valve and monitoring an anesthetic agent percentage, comparing the anesthetic agent percentage to a threshold calibration anesthetic agent percentage, and determining a calibrated liftoff current in response to the anesthetic agent percentage being greater than the threshold calibration anesthetic agent percentage. In a fifth example of the method, optionally including one or more or each of the first through fourth examples, the method further comprises: supplying a calibrated liftoff current to the proportional valve during the therapy mode subsequent calibrating the proportional valve, wherein the calibrated liftoff current is an end result of the calibrating and is supplied to the proportional valve during an opening of the proportional valve from the closed position. In a sixth example of the method, optionally including one or more or each of the first through fifth examples, the calibrated liftoff current is equal to a highest current supplied to the proportional valve, during the calibrating, minus a threshold value associated with an anesthetic sensor delay. In a seventh example of the method, optionally including one or more or each of the first through sixth examples, the method further comprises: terminating calibration of the proportional valve in response to a time elapsing or a current applied to the proportional valve exceeding a threshold calibration current.

The disclosure also provides support for a system for an anesthetic vaporizer, comprising: a mixing gas line coupling to a mixing manifold, an outlet line coupling a vaporization chamber to the mixing manifold, a proportional valve arranged in the outlet line between the vaporization chamber and the mixing manifold, and a controller with instructions stored on non-transitory memory thereof that when executed cause the controller to: calibrate a liftoff current of the proportional valve during a calibration mode following a standby mode and prior to a therapy mode via monitoring an anesthetic percentage as the proportional valve is opened. In a first example of the system, the calibration mode comprises providing a previous liftoff current to the proportional valve minus a determined value and comparing the anesthetic percentage to a threshold percentage. In a second example of the system, optionally including the first example, the system further comprises: increasing current supplied to the proportional valve until the anesthetic percentage exceeds the threshold percentage. In a third example of the system, optionally including one or both of the first and second examples, the instructions further cause the controller to record a calibrated liftoff current equal to a liftoff current at which the anesthetic percentage exceeded the threshold percentage minus a sensor delay value, wherein the calibrated liftoff current is used during the therapy mode subsequent the calibration mode. In a fourth example of the system, optionally including one or more or each of the first through third examples, the instructions further cause the controller to terminate the calibration mode in response to a current supplied to the proportional valve exceeding a threshold calibration current. In a fifth example of the system, optionally including one or more or each of the first through fourth examples, the instructions further cause the controller to terminate the calibration mode in response to a duration of the calibration mode exceeding a threshold duration. In a sixth example of the system, optionally including one or more or each of the first through fifth examples, the controller is a first controller, further comprising a second controller that is unused during the calibration mode and used during the therapy mode.

The disclosure also provides support for a method, comprising: calibrating a liftoff current of a proportional valve of an anesthetic vaporizer following a standby mode and prior to a therapy mode via only a first controller, and operating the proportional valve based on a calibrated liftoff current during the therapy mode with the first controller and a second controller. In a first example of the method, the first controller is a current controller. In a second example of the method, optionally including the first example, the second controller is a concentration controller. In a third example of the method, optionally including one or both of the first and second examples, calibrating the liftoff current includes initially providing less than a previous liftoff current to the proportional valve and increasing a current supplied to the proportional valve stepwise until an anesthetic agent percentage is greater than a threshold anesthetic agent percentage. In a fourth example of the method, optionally including one or more or each of the first through third examples, the calibrated liftoff current is less than a highest current supplied to the proportional valve during the calibrating.

FIGS. 1 and 12 show example configurations with relative positioning of the various components. If shown directly contacting each other, or directly coupled, then such elements may be referred to as directly contacting or directly coupled, respectively, at least in one example. Similarly, elements shown contiguous or adjacent to one another may be contiguous or adjacent to each other, respectively, at least in one example. As an example, components laying in face-sharing contact with each other may be referred to as in face-sharing contact. As another example, elements positioned apart from each other with only a space there-between and no other components may be referred to as such, in at least one example. As yet another example, elements shown above/below one another, at opposite sides to one another, or to the left/right of one another may be referred to as such, relative to one another. Further, as shown in the figures, a topmost element or point of element may be referred to as a "top" of the component and a bottommost element or point of the element may be referred to as a "bottom" of the component, in at least one example. As used herein, top/bottom, upper/lower, above/below, may be relative to a vertical axis of the figures and used to describe positioning of elements of the figures relative to one another. As such, elements shown above other elements are positioned vertically above the other elements, in one example. As yet another example, shapes of the elements depicted within the figures may be referred to as having those shapes (e.g., such as being circular, straight, planar, curved, rounded, chamfered, angled, or the like). Further, elements shown intersecting one another may be referred to as intersecting elements or intersecting one another, in at least one example. Further still, an element shown within another element or shown outside of another element may be referred as such, in one example.

As used herein, an element or step recited in the singular and preceded with the word "a" or "an" should be understood as not excluding plural of said elements or steps, unless such exclusion is explicitly stated. Furthermore, references to "one embodiment" of the present invention are not intended to be interpreted as excluding the existence of additional embodiments that also incorporate the recited features. Moreover, unless explicitly stated to the contrary, embodiments "comprising," "including," or "having" an element or a plurality of elements having a particular property may include additional such elements not having that property. The terms "including" and "in which" are used as the plain-language equivalents of the respective terms "comprising" and "wherein." Moreover, the terms "first," "second," and "third," etc. are used merely as labels, and are not intended to impose numerical requirements or a particular positional order on their objects.

This written description uses examples to disclose the invention, including the best mode, and also to enable a person of ordinary skill in the relevant art to practice the invention, including making and using any devices or systems and performing any incorporated methods. The patentable scope of the invention is defined by the claims, and may include other examples that occur to those of ordinary skill in the art. Such other examples are intended to be within the scope of the claims if they have structural elements that do not differ from the literal language of the claims, or if they include equivalent structural elements with insubstantial differences from the literal languages of the claims.

## Claims

1. A method, comprising:
calibrating a liftoff current of a proportional valve arranged between a vaporization chamber and a mixing manifold of an anesthetic vaporizer via a controller following a standby mode and prior to a therapy mode, wherein the liftoff current is a current supplied to actuate the proportional valve from a closed position to an open position.

2. The method of claim 1, wherein the controller is a first controller, further comprising a second controller that is unused during the calibrating.

3. The method of claim 2, wherein the first controller and the second controller are used during the therapy mode.

4. The method of claim 1, wherein the calibrating occurs only in response to the therapy mode being requested during the standby mode.

5. The method of claim 1, wherein calibrating the liftoff current further comprises initially supplying a previous liftoff current minus a determined value to the proportional valve and monitoring an anesthetic agent percentage;
comparing the anesthetic agent percentage to a threshold calibration anesthetic agent percentage; and
determining a calibrated liftoff current in response to the anesthetic agent percentage being greater than the threshold calibration anesthetic agent percentage.

6. The method of claim 1, further comprising supplying a calibrated liftoff current to the proportional valve during the therapy mode subsequent calibrating the proportional valve, wherein the calibrated liftoff current is an end result of the calibrating and is supplied to the proportional valve during an opening of the proportional valve from the closed position.

7. The method of claim 6, wherein the calibrated liftoff current is equal to a highest current supplied to the proportional valve, during the calibrating, minus a threshold value associated with an anesthetic sensor delay.

8. The method of claim 1, further comprising terminating calibration of the proportional valve in response to a time elapsing or a current applied to the proportional valve exceeding a threshold calibration current.

9. A system for an anesthetic vaporizer, comprising:
a mixing gas line coupling to a mixing manifold;
an outlet line coupling a vaporization chamber to the mixing manifold;
a proportional valve arranged in the outlet line between the vaporization chamber and the mixing manifold; and
a controller with instructions stored on non-transitory memory thereof that when executed cause the controller to:
calibrate a liftoff current of the proportional valve during a calibration mode following a standby mode and prior to a therapy mode via monitoring an anesthetic percentage as the proportional valve is opened.

10. The system of claim 9, wherein the calibration mode comprises providing a previous liftoff current to the proportional valve minus a determined value and comparing the anesthetic percentage to a threshold percentage.

11. The system of claim 10, further comprising increasing current supplied to the proportional valve until the anesthetic percentage exceeds the threshold percentage.

12. The system of claim 11, wherein the instructions further cause the controller to record a calibrated liftoff current equal to a liftoff current at which the anesthetic percentage exceeded the threshold percentage minus a sensor delay value, wherein the calibrated liftoff current is used during the therapy mode subsequent the calibration mode.

13. The system of claim 9, wherein the instructions further cause the controller to terminate the calibration mode in response to a current supplied to the proportional valve exceeding a threshold calibration current.

14. The system of claim 9, wherein the instructions further cause the controller to terminate the calibration mode in response to a duration of the calibration mode exceeding a threshold duration.

15. The system of claim 9, wherein the controller is a first controller, further comprising a second controller that is unused during the calibration mode and used during the therapy mode.
